# EUROPEAN PATENT APPLICATION

(11) **EP 4 434 965 A1**
(43) Date of publication of application: **25.09.2024**
(21) Application number: 22932813.3
(22) Date of filing: 13.04.2022
(51) Int. Cl.: C07D 207/20

(54) **METHOD FOR CONTINUOUSLY SYNTHESIZING N-BOC-2,5-DIHYDROPYRROLE**

(30) Priority: 21.03.2022 CN 202210274721
(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); JIAO, Jianye, Tianjin 300457 (CN); YAN, Honglei, Tianjin 300457 (CN); ZHANG, Tao, Tianjin 300457 (CN); LIU, Bin, Tianjin 300457 (CN); LIN, Han, Tianjin 300457 (CN); XIONG, Zhengchang, Tianjin 300457 (CN)
(74) Representative: Berggren Oy
(86) International application number: PCT/CN2022/086690
(87) International publication number: WO 2023/178754

(57) **Abstract**

The present disclosure discloses a method for continuously synthesizing N-Boc-2,5-dihydropyrrole. The method includes the following steps: cis-1,4 dichloro-2 butene is dissolved in an organic solvent, to prepare solution A; urotropine is dissolved in an organic solvent, to prepare solution B; the solution A and solution B are fed into a first continuous reaction device; a product overflow system of the first continuous reaction device is transferred to a second continuous reaction device, and concentrated hydrochloric acid is fed into the same; a product overflow system of the second continuous reaction device is transferred to a third continuous reaction device, and potassium carbonate is fed into the same; a product overflow system of the third continuous reaction device is transferred to a fourth continuous reaction device, and Boc acid anhydride solution is fed into the same; and a product of the fourth continuous reaction device is subjected to continuous solid-liquid separation, concentration and distillation, to obtain N-Boc-2,5-dihydropyrrole. By using technical solution of the present disclosure, the route involving high-risk materials is avoided, and the production capacity and yield are improved in a continuous manner.

## Description

### Technical Field

The present disclosure relates to the field of pharmaceutical and chemical engineering, in particular to a method for continuously synthesizing N-Boc-2,5-dihydropyrrole.

### Background

N-Boc-2,5-dihydropyrrole (also known as N-Boc-3-pyrroline, cas: 73286-70-1) is a raw material needed widely in pharmaceutical chemistry and an important heterocyclic building block. However, due to its longer synthesis route and complex process, the production cost is relatively high. Therefore, improving the process and reducing its production cost are very valuable and necessary.

At present, the main synthesis routes of N-Boc-2,5-dihydropyrrole include the following routes reported in documents: Practical One-Pot and Large-Scale Synthesis of N-(tert-Butyloxycarbonyl)-3-pyrroline (Organic Process Research & Development 2009,13,638-640):

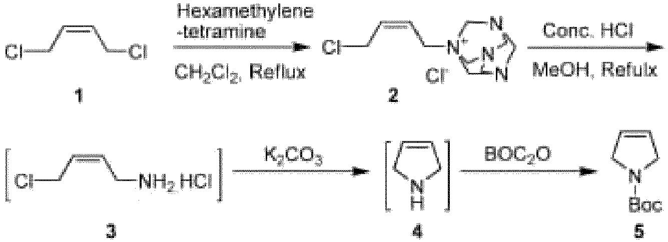

Herein, hexamethylenetetramine is cyclohexene tetramine, Reflux is reflux, Conc.HCl is concentrated hydrochloric acid.

This route avoids dangerous reducing reagents, but no subsequent cyclopropanation researches are conducted. In addition, the report conducts 1000 L batch amplification and finds oxidation and coupling impurities, which are attributed to multi-step post-treatment and resulted in unstable intermediate degradation, and the yield is only 85%. However, this document does not propose a subsequent solution scheme.

Ring-Closing Metathesis in Methanol and Water , Thomas A.Kirkland (J.Org.Chem.1998,63,9904-9909) reports that direct synthesis is achieved by an olefin metathesis method using a Grubbs reagent, and the route is the shortest. But this method has a relatively high raw material cost, and preparing the Grubbs reagent requires ruthenium chloride and expensive ligands, which does not have a cost advantage. In addition, the reaction may generate ethylene, it is a flammable gas, and the enlarged process is low in safety. Its synthesis route is as follows:

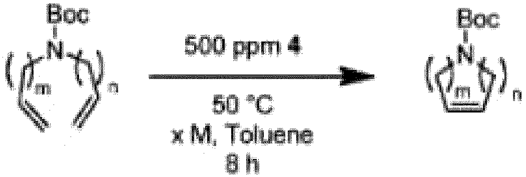

Herein, Tolene is toluene.

In addition, there are other different routes reported, but all have the problem of relatively high raw material costs.

### Summary

The present disclosure aims to provide a method for continuously synthesizing N-Boc-2,5-dihydropyrrole, as to reduce the production cost and improve the yield.

In order to achieve the above purpose, according to one aspect of the present disclosure, a method for continuously synthesizing N-Boc-2,5-dihydropyrrole is provided. The method includes the following steps: cis-1,4 dichloro-2 butene is dissolved in an organic solvent, to prepare solution A; urotropine is dissolved in an organic solvent, to prepare solution B; the solution A and solution B are fed into a first continuous reaction device by using a continuous feeding system; a product overflow system of the first continuous reaction device is transferred to a second continuous reaction device, and concentrated hydrochloric acid is fed into the second continuous reaction device; a product overflow system of the second continuous reaction device is transferred to a third continuous reaction device, and potassium carbonate is fed into the third continuous reaction device; a product overflow system of the third continuous reaction device is transferred to a fourth continuous reaction device, and Boc acid anhydride solution is fed into the fourth continuous reaction device; and a product of the fourth continuous reaction device is subjected to continuous solid-liquid separation, concentration and distillation, to obtain N-Boc-2,5-dihydropyrrole.

Further, the first continuous reaction device, the second continuous reaction device, the third continuous reaction device and the fourth continuous reaction device are a continuous stirred tank reactor (CSTR) or a continuous pipeline reactor respectively.

Further, the concentration is performed by using a continuous thin film evaporation device; and preferably, the concentration is performed at 40~50°C.

Further, the organic solvent is selected from one or more of a group consisting of tetrahydrofuran, 2-methyltetrahydrofuran, 1,4-dioxane, ethylene glycol dimethyl ether, diethylene glycol dimethyl ether, benzene, toluene, xylene, N,N-dimethylformamide, N,N-dimethylacetamide, N,N-diethylformamide, N-methylpyrrolidone, dimethyl sulfoxide, acetonitrile, methanol, ethanol, or isopropanol.

Further, the reaction temperature in the first continuous reaction device is 50~70°C, and the retention time is 1-4 h, preferably 2 h.

Further, the reaction temperature in the second continuous reaction device is 50~60°C, and the retention time is 10-60 min, preferably 30 min.

Further, the reaction temperature in the third continuous reaction device is 50~60°C, and the retention time is 10-60 min, preferably 30 min.

Further, the reaction temperature in the fourth continuous reaction device is 20~40°C, and the retention time is 0.5-4 h, preferably 1 h.

Further, the mass volume percentage concentration of the cis-1,4 dichloro-2 butene in the solution A is 10-20.2%, and the mass volume percentage concentration of the urotropine in the solution B is 21.8%; and the feeding mass ratio of the solution A and the solution B is 1:1.0~2.0.

Further, the ratio of Boc acid anhydride in the Boc acid anhydride solution is 20-80%.

By applying technical solutions of the present disclosure, the route involving high-risk materials is avoided, a multi-step reaction combined production process is achieved in a continuous manner, the safety risks of traditional batch processes are reduced, the material usage amount is reduced, the damage of unstable intermediates is reduced, the multi-step post-treatment is omitted, and the production capacity and yield are improved.

### Detailed Description of the Embodiments

It should be noted that, in the case without conflicting, embodiments in the present application and features in the embodiments may be combined with each other. The present disclosure is described in detail below in combination with the embodiments.

In response to technical problems of longer synthesis routes, complex processes, high costs, and unstable intermediates in the synthesis of N-Boc-2,5-dihydropyrrole in existing technologies, the present disclosure proposes the following technical schemes.

The route of the present disclosure:

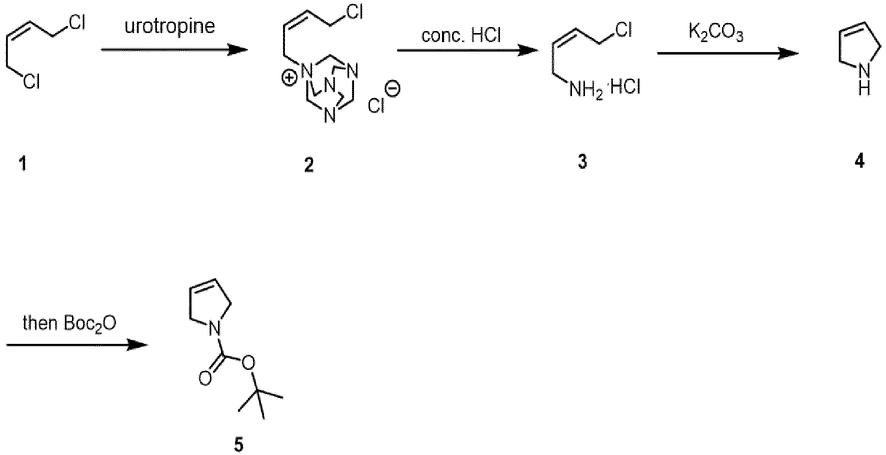

The present disclosure adopts a route that avoids high-risk materials and uses a process that may achieve continuous whole process, with the goal of minimizing the intermediate damage, to directly synthesize a highly stable intermediate N-Boc-2,5-dihydropyrrole. Finally, an efficient and high-yield process is developed.

According to a typical implementation mode of the present disclosure, a method for continuously synthesizing N-Boc-2,5-dihydropyrrole is provided. The method includes the following steps: cis-1,4 dichloro-2 butene is dissolved in an organic solvent, to prepare solution A; urotropine is dissolved in an organic solvent, to prepare solution B; the solution A and solution B are fed into a first continuous reaction device by using a continuous feeding system; a product overflow system of the first continuous reaction device is transferred to a second continuous reaction device, and concentrated hydrochloric acid is fed into the second continuous reaction device; a product overflow system of the second continuous reaction device is transferred to a third continuous reaction device, and potassium carbonate is fed into the third continuous reaction device; a product overflow system of the third continuous reaction device is transferred to a fourth continuous reaction device, and Boc acid anhydride solution is fed into the fourth continuous reaction device; and a product of the fourth continuous reaction device is subjected to continuous solid-liquid separation, concentration and distillation, to obtain N-Boc-2,5-dihydropyrrole.

By applying technical solution of the present disclosure, the route involving high-risk materials is avoided, a multi-step reaction combined production process is achieved in a continuous manner, the safety risks of traditional batch processes are reduced, the material usage amount is reduced, the damage of unstable intermediates is reduced, the multi-step post-treatment is omitted, and the production capacity and yield are improved.

Preferably, the first continuous reaction device, the second continuous reaction device, the third continuous reaction device and the fourth continuous reaction device are a CSTR or a continuous pipeline reactor respectively. Due to the characteristics of the intermediate in this route that is not able to exist stably for a long time, the continuous technology reduces the risks of damage and deterioration, immediately transfers it to the next stage of the reaction while being generated, and ultimately a stable target product is formed. In order to make the process more beneficial to industrial production, a continuous thin film distillation device is used for concentration; and preferably, the concentrate is performed at 40~50°C. The thin film concentration has a negative pressure effect, and low-temperature concentration may be performed, to prevent the product from being damaged by the high temperature. At the same time, the thin film concentration is a continuous technology that reduces the heating time of the product and is also an important factor in improving the yield.

According to a typical implementation mode of the present disclosure, the organic solvent is selected from one or more of a group consisting of tetrahydrofuran, 2-methyltetrahydrofuran, 1,4-dioxane, ethylene glycol dimethyl ether, diethylene glycol dimethyl ether, benzene, toluene, xylene, N,N-dimethylformamide, N,N-dimethylacetamide, N,N-diethylformamide, N-methylpyrrolidone, dimethyl sulfoxide, acetonitrile, methanol, ethanol, and isopropanol, and preferably, the organic solvent is the methanol, because the methanol has better solubility in both aqueous and organic phases, the reaction rate between the two phases is promoted. In addition, the methanol is cheap, easy to concentrate, and has simple post-treatment.

Preferably, in an embodiment of the present disclosure, the reaction temperature in the first continuous reaction device is 50~70°C, and the retention time is 2 h. The reaction temperature in the second continuous reaction device is 50~60°C, and the retention time is 30 min. The reaction temperature in the third continuous reaction device is 50~60°C, and the retention time is 30 min. The reaction temperature in the fourth continuous reaction device is 20~40°C, and the retention time is 1 h. More preferably, the mass volume percentage concentration of cis-1 ,4 dichloro-2 butene in the solution A is 10-20.2%, and the mass volume percentage concentration of the urotropine in the solution B is 21.8%; the feeding mass ratio of the solution A and the solution B is 1:1.0~2.0; and the ratio of the Boc acid anhydride in the Boc acid anhydride solution is 20-80%.

The beneficial effects of the present disclosure are further described below in combination with embodiments.

### Embodiment 1

2 kg of cis-1,4 dichloro-2 butene was dissolved in 10 L of methanol, to prepare solution A. 2.2 kg of urotropine was dissolved in 10 L of methanol, to obtain solution B. Then, a continuous feeding system was used to feed the two solutions into a first CSTR at feeding rates of 10.0 g/min and 10.2 g/min respectively. The temperature of the first CSTR was controlled at 50~70°C, and the retention time was 2 h. Then an overflow system was transferred to a second CSTR, and concentrated hydrochloric acid was fed into the same at a speed of 6.2 g/min, the temperature of the second CSTR was controlled at 50~60°C, and the retention time was 30 min. Then, the system was overflowed to a third CSTR, and a continuous solid feeder was used to feed potassium carbonate into the third CSTR at a speed of 10.5 g/min, the temperature was controlled at 50~60°C, and the retention time was 30 min. Then, the overflow system was transferred to a fourth CSTR, and methanol solution of Boc acid anhydride (3.49 kg of Boc acid anhydride was dissolved in 2 L of methanol) was added at a feeding rate of 5.2 g/min, the temperature was controlled at 20~40°C, and the retention time was 1 h. The reaction system was subjected to continuous solid-liquid separation, and mother solution was concentrated with a thin film. At 40~50°C, it was concentrated into a 2 V crude product. Then, after continuous distillation, a pure intermediate 5 was obtained, the mass was 2.53 kg, and the yield was 95%.

### Embodiment 2

2 kg of cis-1,4 dichloro-2 butene was dissolved in 10 L of methanol, to prepare solution A. 2.2 kg of urotropine was dissolved in 10 L of methanol, to obtain solution B. Then, a continuous feeding system was used to feed the two solutions into a first CSTR at feeding rates of 10.0 g/min and 10.2 g/min respectively. The temperature of the first CSTR was controlled at 50~70°C, and the retention time was 1 h. Then an overflow system was transferred to a second CSTR, and concentrated hydrochloric acid was fed into the same at a speed of 6.2 g/min, the temperature of the second CSTR was controlled at 50~60°C, and the retention time was 10 min. Then, the system was overflowed to a third CSTR, and a continuous solid feeder was used to feed potassium carbonate into the third CSTR at a speed of 10.5 g/min, the temperature was controlled at 50~60°C, and the retention time was 10 min. Then, the overflow system was transferred to a fourth CSTR, and methanol solution of Boc acid anhydride (3.49 kg of Boc acid anhydride was dissolved in 2 L of methanol) was added at a feeding rate of 5.2 g/min, the temperature was controlled at 20~40°C, and the retention time was 0.5 h. The reaction system was subjected to continuous solid-liquid separation, and mother solution was concentrated with a thin film. At 40~50°C, it was concentrated into a 2 V crude product. Then, after continuous distillation, a pure intermediate 5 was obtained, the mass was 2.4 kg, and the yield was 90%.

### Embodiment 3

2 kg of cis-1,4 dichloro-2 butene was dissolved in 10 L of methanol, to prepare solution A. 2.2 kg of urotropine was dissolved in 10 L of methanol, to obtain solution B. Then, a continuous feeding system was used to feed the two solutions into a first CSTR at feeding rates of 10.0 g/min and 10.2 g/min respectively. The temperature of the first CSTR was controlled at 50~70°C, and the retention time was 4 h. Then an overflow system was transferred to a second CSTR, and concentrated hydrochloric acid was fed into the same at a speed of 6.2 g/min, the temperature of the second CSTR was controlled at 50~60°C, and the retention time was 60 min. Then, the system was overflowed to a third CSTR, and a continuous solid feeder was used to feed potassium carbonate into the third CSTR at a speed of 10.5 g/min, the temperature was controlled at 50~60°C, and the retention time was 60 min. Then, the overflow system was transferred to a fourth CSTR, and methanol solution of Boc acid anhydride (3.49 kg of Boc acid anhydride was dissolved in 2 L of methanol) was added at a feeding rate of 5.2 g/min, the temperature was controlled at 20~40°C, and the retention time was 4 h. The reaction system was subjected to continuous solid-liquid separation, and mother solution was concentrated with a thin film. At 40~50°C, it was concentrated into a 2 V crude product. Then, after continuous distillation, a pure intermediate 5 was obtained, the mass was 2.51 kg, and the yield was 94%.

### Embodiment 4

2 kg of cis-1,4 dichloro-2 butene was dissolved in 10 L of methanol, to prepare solution A. 2.2 kg of urotropine was dissolved in 10 L of methanol, to obtain solution B. Then, a continuous feeding system was used to feed the two solutions into a first CSTR at feeding rates of 10.0 g/min and 10.2 g/min respectively. The temperature of the first CSTR was controlled at 50~70°C, and the retention time was 2 h. Then an overflow system was transferred to a second CSTR, and concentrated hydrochloric acid was fed into the same at a speed of 6.2 g/min, the temperature of the second CSTR was controlled at 50~60°C, and the retention time was 30 min. Then, the system was overflowed to a third CSTR, and a continuous solid feeder was used to feed potassium carbonate into the third CSTR at a speed of 10.5 g/min, the temperature was controlled at 50~60°C, and the retention time was 30 min. Then, the overflow system was transferred to a fourth CSTR, and methanol solution of Boc acid anhydride (3.49 kg of Boc acid anhydride was dissolved in 2 L of methanol) was added at a feeding rate of 5.2 g/min, the temperature was controlled at 20~40°C, and the retention time was 1 h. The reaction system was subjected to continuous solid-liquid separation, and mother solution was concentrated with a thin film. At 40~50°C, it was concentrated into a 2 V crude product. Then, after continuous distillation, a pure intermediate 5 was obtained, the mass was 2.50 kg, and the yield was 94%.

From the above description, it may be seen that the above embodiments of the present disclosure achieve the following technical effects: by applying the technical schemes of the present disclosure, the route involving high-risk materials is avoided, the multi-step reaction combined production process is achieved in the continuous manner, the safety risks of traditional batch processes are reduced, the material usage amount is reduced, the damage of unstable intermediates is reduced, the multi-step post-treatment is omitted, and the production capacity and yield are improved.

The above are only preferred embodiments of the present disclosure and are not intended to limit the present disclosure. For those skilled in the art, the present disclosure may have various modifications and variations. Any modifications, equivalent replacements, improvements and the like made within the spirit and principles of the present disclosure shall be contained within the scope of protection of the present disclosure.

## Claims

1. A method for continuously synthesizing N-Boc-2,5-dihydropyrrole, comprising the following steps:
dissolving cis-1,4-dichloro-2-butene in an organic solvent, to obtain solution A;
dissolving urotropine in an organic solvent, to obtain solution B;
feeding the solution A and the solution B into a first continuous reaction device by using a continuous feeding system;
transferring a product overflow system of the first continuous reaction device to a second continuous reaction device, and feeding a concentrated hydrochloric acid into the second continuous reaction device at the same time;
transferring a product overflow system of the second continuous reaction device to a third continuous reaction device, and feeding a potassium carbonate into the third continuous reaction device at the same time;
transferring a product overflow system of the third continuous reaction device to a fourth continuous reaction device, and feeding Boc acid anhydride solution into the fourth continuous reaction device at the same time; and
performing continuous solid-liquid separation, concentration and distillation on a product of the fourth continuous reaction device, to obtain the N-Boc-2,5-dihydropyrrole.

2. The method according to claim 1, wherein the first continuous reaction device, the second continuous reaction device, the third continuous reaction device and the fourth continuous reaction device are a continuous stirred tank reactor (CSTR) or a continuous pipeline reactor respectively.

3. The method according to claim 1, wherein the concentration is performed by using a continuous thin film evaporation device.

4. The method according to claim 1, wherein the concentration is performed at 40~50°C.

5. The method according to claim 1, wherein the organic solvent is selected from one or more of a group consisting of tetrahydrofuran, 2-methyltetrahydrofuran, 1,4-dioxane, ethylene glycol dimethyl ether, diethylene glycol dimethyl ether, benzene, toluene, xylene, N,N-dimethylformamide, N,N-dimethylacetamide, N,N-diethylformamide, N-methylpyrrolidone, dimethyl sulfoxide, acetonitrile, methanol, ethanol, or isopropanol.

6. The method according to any one of claims 1 to 4, wherein the reaction temperature in the first continuous reaction device is 50~70°C, and the retention time is 1-4 h.

7. The method according to claim 6, wherein the retention time in the first continuous reaction device is 2 h.

8. The method according to any one of claims 1 to 4, wherein the reaction temperature in the second continuous reaction device is 50~60°C, and the retention time is 10-60 min.

9. The method according to claim 8, wherein the retention time in the second continuous reaction device is 30 min.

10. The method according to any one of claims 1 to 4, wherein the reaction temperature in the third continuous reaction device is 50~60°C, and the retention time is 10-60 min.

11. The method according to claim 10, wherein the retention time in the third continuous reaction device is 30 min.

12. The method according to any one of claims 1 to 4, wherein the reaction temperature in the fourth continuous reaction device is 20~40°C, and the retention time is 0.5-4 h.

13. The method according to claim 10, wherein the retention time in the fourth continuous reaction device is 1 h.

14. The method according to claim 1, wherein the concentration of the cis-1,4-dichloro-2-butene in the solution A is 10-20.2%, and the concentration of the urotropine in the solution B is 21.8%; and the feeding ratio of the solution A and the solution B is 1:1.0-2.0.

15. The method according to claim 1, wherein the ratio of Boc acid anhydride in the Boc acid anhydride solution is 20-80%.
